# EUROPEAN PATENT APPLICATION

(11) **EP 1 489 178 A1**
(43) Date of publication of application: **22.12.2004**
(21) Application number: 03710429.6
(22) Date of filing: 19.03.2003
(51) Int. Cl.: C12N 15/12, C12N 15/63, C12N 1/19, C12N 1/21, C12N 5/10, C07K 14/47, C07K 16/18, C12P 21/02, C12Q 1/02, C12Q 1/68, A61K 35/76, A61K 38/00, A61K 39/395, A61K 45/00, A61K 48/00, A61P 25/00, G01N 33/15, G01N 33/50, G01N 33/53, G01N 33/566

(54) **POLYPEPTIDES BINDING TO HUMAN SYNTAXIN 1A**

(30) Priority: 19.03.2002 JP 2002075551
(71) Applicant: DAIICHI PHARMACEUTICAL CO., LTD., Chuo-ku, Tokyo 103-8234 (JP)
(72) Inventor: YOKOTA, Hiroshi, c/o DAIICHI PHARM. CO., LTD, Shimotsuga-gun, Tokyo 329-0512 (JP); SATOH, Kazuki, c/o DAIICHI PHARM. CO., LTD, Shimotsuga-gun, Tokyo 329-0512 (JP)
(74) Representative: Forstmeyer, Dietmar, Dr. rer. nat., Dipl.-Chem.
(86) International application number: PCT/JP2003/003308
(87) International publication number: WO 2003/078633

(57) **Abstract**

There is provided a polypeptide that binds to syntaxin-1a and comprises the amino acid sequence shown in SEQ ID NO: 1 in the Sequence Listing, or a polypeptide having a homology of at least approximately 90% to the amino acid sequence that binds to syntaxin-1a, a partial peptide thereof, and a polynucleotide encoding any of these polypeptides or the complementary strand thereof, as well as an inhibitor, an antagonist, and an activator of physiological activity of any of the polypeptides. Further, a pharmaceutical composition that utilizes these is provided. There is also provided a method for producing the polypeptides or the peptide by genetic engineering techniques, a method for identifying the inhibitor, antagonist, or activator, and a method and a kit for diagnosing a disease with which the polypeptides are associated.

## Description

### TECHNICAL FIELD

The present invention relates to a polypeptide that has a vesicle-associated membrane protein (hereinafter also referred to as "VAMP")-homologous region and binds to syntaxin-1a, as well as a peptide having a part of the polypeptide. The present invention also relates to a polypeptide or peptide that promotes the formation of a complex comprising human syntaxin-1a, 25kDa synaptosomal-associated protein (hereinafter also referred to as "SNAP-25") and synaptotagmin, wherein the complex participates in the fusion of synaptic vesicles with presynaptic membranes. The present invention further relates to a regulator of exocytosis that results from the fusion of synaptic vesicles with presynaptic membranes, wherein the regulator contains the polypeptide or the peptide and acts by a binding function to human syntaxin-1a. The present invention also relates to the following: a polynucleotide comprising a nucleotide sequence encoding the polypeptide or peptide, or the complementary sequence thereof; a recombinant vector containing the polynucleotide; or a transformant into which the recombinant vector is transfected. The present invention further relates to the following: an antibody against the polypeptide or peptide; a compound having an ability to interact with the polypeptide, peptide, or the polynucleotide; a pharmaceutical composition containing one or more of these substances; or a pharmaceutical composition that has an action to control the release of a neurotransmitter. The present invention further relates to a method for controlling the release of a neurotransmitter by controlling the binding of the polypeptide to human syntaxin-1a, and to a method for preventing and/or treating diseases that are caused by the abnormal release of neurotransmitters. The present invention still further relates to a measurement means for diagnosis that comprises measuring the polypeptide or peptide or the polynucleotide, a method for producing the polypeptide or peptide using the transformant or the recombinant vector, a method for identifying a compound that acts on the binding of the polypeptide to human syntaxin-1a or a compound that regulates expression of the polynucleotide, and a reagent kit to be used with the measurement means and the identification method.

### BACKGROUND ART

Syntaxin-1, which is mainly present in the brain, is a protein that relates to a release mechanism for neurotransmitters. The isoforms, syntaxin 1a and 1b, are known. Upon release of neurotransmitters (exocytosis), syntaxin 1 forms a complex with synaptosomal-associated 25kDa protein (SNAP-25), vesicle-associated membrane protein (VAMP, also called "synaptobrevin") and synaptotagmin/p65, and participates in the process of docking and/or fusion of synaptic vesicles with presynaptic membranes. In the complex, VAMP is localized in synaptic vesicles and functions as a v-SNARE (vesicle-soluble N-ethylmaleimide-sensitive factor attachment receptor), and syntaxin 1 and SNAP-25 are mainly localized in presynaptic membranes and function as a t-SNARE (target-soluble N-ethylmaleimide-sensitive factor attachment receptor). SNARE (soluble N-ethylmaleimide-sensitive factor attachment receptor) formation is inhibited by MUNC-18, which binds to syntaxin 1.

The binding of syntaxin 1a, which is a principal molecule among the syntaxins, to m-tomosyn may be significant in the following respects. Among the many proteins involved in docking and membrane fusion of synaptic vesicles upon exocytosis of neurotransmitters, syntaxin is a t-SNARE that is considered to be the molecule fulfilling the main role therein. Syntaxin 1 combines with SNAP-25, which is another t-SNARE, as well as with VAMP and synaptotagmin, which are two kinds of v-SNARE, to form a 7S (S: sedimentation coefficient) complex, and consequently the synaptotagmin therein is replaced by α-SNAP (soluble NSF-attachment protein) and NSF (N-ethylmaleimide-sensitive factor) to form a 20S complex, which is considered to be extremely important for the start of the docking and membrane fusion mentioned above.

Fujita et al. analyzed the glycerol gradient fractions of rat brain LP2 fraction, using antibodies against each constitutive protein of SNARE (such as anti-tomosyn antibody), for the constitutive proteins in each fraction. Their findings revealed that m-tomosyn forms a 10S complex with SNAP-25 and synaptotagmin. They also exhibited data suggesting that m-tomosyn dissociates MUNC-18 from a complex comprising syntaxin and MUNC-18 to bind to the syntaxin (non-patent document 1). Further, high level expression of m-tomosyn in a PC12 cell reduced the amount of calcium-dependent exocytosis by 30% (non-patent document 1). MUNC-18 and synaptophysin are considered to suppressively control the formation of SNARE by binding to syntaxin and VAMP, respectively. It is believed that m-tomosyn replaces MUNC-18 to bind to syntaxin-1a due to its having a higher affinity than MUNC-18, and thereby forms a 10S complex, which then sequentially changes to a 7S complex and then a 20S complex through steps of some kind, and finally resulting in the fusion of synaptic vesicles with presynaptic membranes.

In short, there is a possibility that m-tomosyn, which is a protein having high binding affinity to syntaxin that is believed to play a central role in the fusion of synaptic vesicle with presynaptic membrane, plays a highly important role in the docking and fusion of synaptic vesicles.

The references cited herein are listed below.
Patent document 1: Method for producing recombinant baculovirus expression vector; Japanese Patent No. 2129487.
Patent document 2: Method for producing polypeptide; Japanese Patent No. 2644447.
Non-patent document 1: Fujita, Y. et al., Tomosyn: a syntaxin-1-binding protein that forms a novel complex in the neurotransmitter release process; Neuron, (1998) 20, p.905-915.
Non-patent document 2: Yokoyama, S. et al., Three splicing variants of tomosyn and identification of their syntaxin-binding region; Biochemical and Biophysical Research Communications, (1999) 256, p.218-222.
Non-patent document 3: Masuda, E. S. et al., Tomosyn binds t-SNARE proteins via a VAMP-like coiled coil; Neuron, (1998) 21, p.479-480.
Non patent document 4: Lehman, K. et al., Yeast homologue of tomosyn and Lethal giant larvae function in exocytosis and are associated with the plasma membrane SNARE; Sec. 9, Journal of Cell Biology, (1999) 146, p.125-140.
Non-patent document 5: Sambrook et al., "Molecular Cloning, A Laboratory Manual, second edition" Cold Spring Harbor Laboratory (1989).
Non-patent document 6: Masami Muramatu, "Lab Manual of Genetic Engineering", MARUZEN Co., Ltd. (1988).
Non-patent document 7: Ehrlich, H. E et al., "PCR Technology, Theory and Application of DNA Amplification," Stockton Press (1989).
Non-patent document 8: Ulmer, K. M., Science, (1983) 219, p.666-671.
Non-patent document 9: Nature, (1957) 179, p.160-161.
Non-patent document 10: Hata, Y. et al., Synaptic vesicle fusion complex contains unc-18 homologue bound to syntaxin (1993), Nature, (1993) 366, p.347-351.
Non-patent document 11: Hata, Y. et al., A novel ubiquitous form of Munc-18 interacts with multiple syntaxins, Journal of Biological Chemistry, (1995) 270, pp. 13022-13028.
Non-patent document 12: Kee, Y. et al., Distinct domains of syntaxin are required for synaptic vesicle complex formation and dissociation, Neuron, (1995) 14, p.991-998.
Non-patent document 13: Kee, Y. et al., Localization of synaptotagmin-binding domain on syntaxin, Journal of Neuroscience, (1996) 16, p.1975-1981.

### DISCLOSURE OF THE INVENTION

In consideration of the facts described above, it is an object of the present invention to find and provide a polypeptide or peptide that binds to human syntaxin-1a. Another object of the present invention is to provide a polynucleotide encoding the polypeptide or peptide that binds to human syntaxin-1a and to provide a method for producing the polypeptide or peptide that binds to human syntaxin-1a using genetic engineering techniques. A further object of the present invention is to provide an antibody for the polypeptide or peptide that binds to human syntaxin-1a, and an inhibiter or a stabilizer of the binding of human syntaxin-1a with the polypeptide or peptide that binds to human syntaxin-1a. A still further object of the present invention is to identify, using the aforementioned method and substances, an antibody for the polypeptide or peptide that binds to human syntaxin-1a, as well as an inhibitor or stabilizer of the binding of human syntaxin-1a to a polypeptide or peptide that binds to human syntaxin-1a, and to provide a pharmaceutical composition using the aforementioned substances, as well as a measurement method and a reagent kit for diagnosis.

The present inventors have conducted concentrated studies to achieve the objects mentioned above, and found that a part of the KIAA1006 clone (GenBank accession number: AB023223) that is disclosed in the human long chain cDNA analysis information database of the Kazusa DNA Research Institute has high homology to the VAMP homologous region of rat m-tomosyn. More specifically, they performed a homology search in the human long chain cDNA analysis information database of the Kazusa DNA Research Institute with respect to the VAMP homologous region that is indicated as being important in binding to syntaxin-1a in rat m-tomosyn, and selected KIAA 1006. The present inventors then determined the full-length open reading frame (ORF) of KIAA1006 gene and carried out the expression of the gene in a gene expression system using an insect cell to obtain a polypeptide that is the gene product encoded by the gene. Further, they demonstrated that the thus obtained polypeptide binds to human syntaxin-1a. They completed the present invention by providing a pharmaceutical composition for a disease attributable to the polypeptide by regulating the function and physiological effects of the polypeptide, as well as a measurement means for diagnosing the disease.

More specifically, the first aspect of the present invention relates to a polypeptide that binds to human syntaxin-1a, wherein the polypeptide is selected from the group consisting of:
(i) a polypeptide comprising the amino acid sequence shown in SEQ ID NO: 1 in the Sequence Listing;
(ii) a polypeptide containing the polypeptide of the above (i);
(iii) a polypeptide having at least 90% homology to the amino acid sequence of the polypeptide of the above (i); and
(iv) a polypeptide having a mutation such as a deletion, substitution, addition or insertion of one or several amino acids with respect to the amino acid sequence of any of the polypeptides of the above (i) to (iii).

Another aspect of the present invention relates to a peptide comprising at least five consecutive amino acid residues in the amino acid sequence shown in SEQ ID NO: 1 in the Sequence Listing.

A further aspect of the present invention relates to the aforementioned polypeptide or peptide, wherein the polypeptide or the peptide promotes the formation of a complex that comprises human syntaxin-1a, synaptosomal-associated 25kDa protein and synaptotagmin, and is involved in the fusion of a synaptic vesicle with a presynaptic membrane.

A further aspect of the present invention relates to a regulator of exocytosis through fusion of a synaptic vesicle with a presynaptic membrane, wherein the regulator comprises the aforementioned polypeptide or peptide and has a binding function to human syntaxin-1a.

A still further aspect of the present invention relates to a polynucleotide comprising a nucleotide sequence encoding the aforementioned polypeptide or peptide, or a complementary sequence thereof.

A further aspect of the present invention relates to a polynucleotide that hybridizes to the aforementioned polynucleotide under stringent conditions.

A still further aspect of the present invention relates to a polynucleotide having at least 15 consecutive nucleotides of the aforementioned polynucleotide.

A further aspect of the present invention relates to a recombinant vector containing the aforementioned polynucleotide.

A further aspect of the present invention relates to the aforementioned recombinant vector, wherein the recombinant vector is a recombinant expression vector.

A still further aspect of the present invention relates to a transformant that is transformed with the aforementioned recombinant vector.

A further aspect of the present invention relates to a method for producing the aforementioned polypeptide or peptide, wherein the method comprises a step of incubating the aforementioned transformant transformed with the aforementioned recombinant vector.

A still further aspect of the present invention relates to an antibody that immunologically recognizes the aforementioned polypeptide or peptide.

A further aspect of the present invention relates to the aforementioned antibody that inhibits the binding of the aforementioned polypeptide or peptide to a protein that interacts with the polypeptide or the peptide.

A further aspect of the present invention relates to the aforementioned antibody, which inhibits the binding of the aforementioned polypeptide or peptide to human syntaxin-1a.

A further aspect of the present invention relates to a method for identifying a compound that inhibits or stabilizes the binding of the aforementioned polypeptide or peptide to a protein that interacts with the polypeptide or the peptide and/or a compound that inhibits or promotes expression of the aforementioned polynucleotide, wherein the method uses at least one member selected from the group consisting of the aforementioned polypeptide or peptide, the aforementioned polynucleotide, the aforementioned vector, the aforementioned transformant and the aforementioned antibody.

A still further aspect of the present invention relates to a method for identifying a compound that inhibits or stabilizes the binding of the aforementioned polypeptide or peptide to a protein that interacts with the polypeptide or peptide, wherein the method comprises steps of: contacting a test substance with the polypeptide or the peptide and a protein that interacts with the polypeptide or peptide, under conditions that allow the binding of the polypeptide or the peptide to a protein that interacts with the polypeptide or peptide; and determining whether the test substance inhibits or stabilizes the binding of the polypeptide or the peptide to a protein that interacts with the polypeptide or peptide, by detecting the presence, absence, or change of a signal generated by binding of the polypeptide or the peptide to a protein that interacts with the polypeptide or peptide.

A further aspect of the present invention relates to a method for identifying a compound that inhibits or stabilizes the binding of the aforementioned polypeptide or peptide to human syntaxin-1a, wherein the method comprises steps of: contacting a test substance with the polypeptide or the peptide and human syntaxin 1a, under conditions that allow the binding of the polypeptide or the peptide to human syntaxin-1a; and determining whether the test substance inhibits or stabilizes the binding of the polypeptide or the peptide to human syntaxin-1a, by detecting the presence, absence, or change of a signal generated by the binding of the polypeptide or the peptide to human syntaxin-1a.

A still further aspect of the present invention relates to a method for identifying a compound that inhibits or promotes expression of any of the aforementioned polynucleotides, wherein the method comprises steps of: contacting a test substance with the polynucleotide under conditions that allow expression of the polynucleotide; and determining whether the test substance inhibits or promotes expression of the polynucleotide by detecting the presence, absence, or change of a signal generated by expression of the polynucleotide.

A further aspect of the present invention relates to a compound that is identified by any of the aforementioned method for identifying a compound.

A further aspect of the present invention relates to a compound that inhibits or stabilizes the binding of the aforementioned polypeptide or peptide to human syntaxin-1a by interacting with the polypeptide or peptide.

A still further aspect of the present invention relates to a compound that inhibits or promotes expression of any of the aforementioned polynucleotides by interacting with the polynucleotide.

A further aspect of the present invention relates to a pharmaceutical composition comprising at least one member selected from the group consisting of the aforementioned polypeptide or peptide, the aforementioned polynucleotide, the aforementioned vector, the aforementioned transformant, the aforementioned antibody, and the aforementioned compound.

A further aspect of the present invention relates to the aforementioned pharmaceutical composition, which has a function to promote or inhibit the release of neurotransmitters.

A further aspect of the present invention relates to the aforementioned pharmaceutical composition, which is used as a preventive and/or therapeutic agent for a disease caused by an abnormal amount of neurotransmitters.

A still further aspect of the present invention relates to a method for promoting or inhibiting the release of neurotransmitters, wherein the method comprises inhibiting or stabilizing the binding of the aforementioned polypeptide to human syntaxin-1a.

A further aspect of the present invention relates to a method for preventing or inhibiting a disease caused by an abnormal amount of neurotransmitters, wherein the method comprises inhibiting or stabilizing the binding of the aforementioned polypeptide to human syntaxin-1 a.

A further aspect of the present invention relates to a method for measurement used in diagnosis of a disease caused by an abnormality in the expression or function of the aforementioned polypeptide, wherein the method comprises conducting an analysis employing (A) a nucleic acid encoding the polypeptide, and/or (B) the polypeptide as a marker.

A further aspect of the present invention relates to a method for measurement used in diagnosis of a disease associated with an abnormal function of neurotransmittion caused by an abnormal amount of the aforementioned polypeptide, wherein the method comprises conducting an analysis employing (A) a nucleic acid encoding the polypeptide and/or (B) the polypeptide as a marker.

A still further aspect of the present invention relates to a reagent kit comprising at least one member selected from the group consisting of the aforementioned polypeptide or peptide, the aforementioned polynucleotide and the aforementioned antibody.

A further aspect of the present invention relates to a reagent kit for use in the aforementioned identification method or the aforementioned measurement method.

### BRIEF DESCRIPTION OF THE DRAWINGS

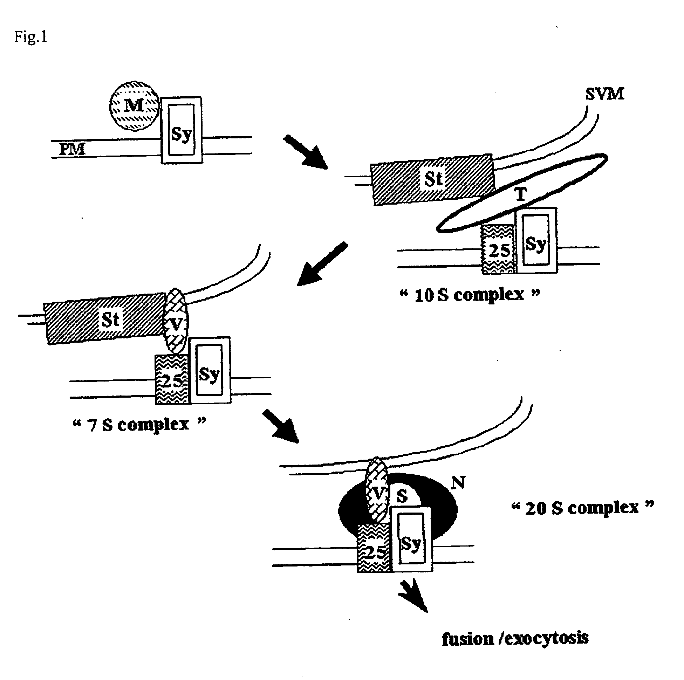

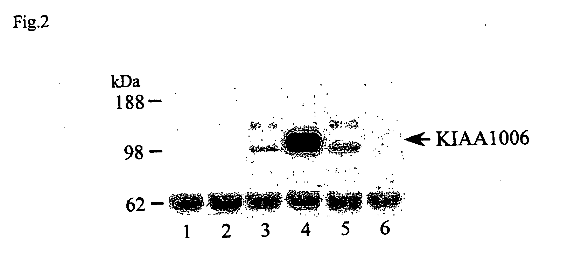

Fig. 1 is a schematic diagram to illustrate that synaptic vesicles fuse with presynaptic membrane in the exocytosis of neurotransmitters through the steps of: 1) replacement of MUNC-18 that is bound to syntaxin with tomosyn, 2) formation of a 10S complex, 3) formation of a 7S complex, and 4) formation of a 20S complex, in this order. The meaning of the each symbol used in Fig. 1 is listed below.
   PM: presynaptic membrane
   SVM: synaptic vesicle membrane
   Sy: syntaxin
   M: MUNC-18
   T: tomosyn
   N: NSF
   S: α-SNAP
   25: SNAP-25
   V: VAMP
   St: synaptotagmin
Fig. 2 shows the binding of the KIAA1006 polypeptide and C-terminal truncated KIAA1006 to syntaxin-1a. The polypeptide of the present invention and C-terminal truncated product, which were expressed and purified according to a method described in Example herein, were subjected to treatment with glutathione S-transferase (GST)-binding glutathione-Sepharose and then syntaxin-1a-GST-binding glutathione-Sepharose according to a method described in the Example hereunder. Fractions that bound to GST-binding glutathione-Sepharose and syntaxin-1a-GST-binding glutathione-Sepharose were eluted with a sample buffer for sodium dodecyl sulphate-polyacrylamide gel electrophoresis (SDS-PAGE), and then analyzed together with a fraction unadsorbed to the GST-binding glutathione-Sepharose by Western blotting using anti-penta histidine (penta His) antibody (Qiagen). In the figure, lanes 1 and 2 show GST-adsorbed fractions, lanes 3 and 5 show GST-unadsorbed fractions, and lanes 4 and 6 show syntaxin-1a-GST-adsorbed fractions, wherein lanes 1, 3 and 4 show the KIAA1006 polypeptide (1-1186AA), and lanes 2, 5 and 6 show the C-terminal truncated product (1-1157.AA) of KIAA1006 polypeptide.

### DETAILED DESCRIPTION OF INVENTION

The present invention claims the benefit of priority of Japanese Patent Application No. 2002-75551, which is incorporated herein by reference.

Technical and scientific terms used herein have the meanings as understood generally by those skilled in the art, unless otherwise defined. Reference is made herein to a variety of methods that are well-known to those skilled in the art. Data from publications and the like that discloses such cited well-known methods are deemed completely incorporated herein in their entirety by reference.

A mode of embodiment of the present invention may be described in more detail hereafter. The following detailed description is illustrative and merely explanatory, and it does not limit the scope of the present invention.

### (Polypeptide or Peptide)

A polypeptide provided according to the present invention is a polypeptide encoded by KIAA1006, which is a gene selected from a long chain cDNA library derived from the human brain (disclosed by the Kazusa DNA Research Institute) by carrying out a homology search for the VAMP homologous region that has been indicated as being involved in the binding of rat m-tomosyn to syntaxin-1a (non-patent document 2 and non-patent document 3). The polypeptide is obtained as a soluble protein by expressing the ORF region of KIAA1006 in a gene expression system using baculovirus and an insect cell, sf9. The full-length ORF of the KIAA1006 gene comprises 3,558 base pairs, and the product of the gene comprises 1,186 amino acid residues. The homology of KIAA1006 to rat m-tomosyn is 57.6% at the nucleotide level and 67.9% at the amino acid level. In the present invention, it was revealed that the polypeptide binds to human syntaxin-1a. Hereinunder, the ORF region of KIAA1006 is also referred to as "KIAA1006 polypeptide."

As described above, KIAA1006 polypeptide exhibits a high homology to rat m-tomosyn, so it is considered to function in a similar manner to m-tomosyn in humans.

In the exocytosis of a neurotransmitter, synaptic vesicles fuse with the presynaptic membrane through the following steps of: 1) replacement of MUNC-18 binding to syntaxin, with tomosyn; 2) formation of a 10S complex; 3) formation of a 7S complex; and 4) formation of a 20S complex; in this order (refer to Fig. 1) (non-patent document 2 and non-patent document 3). The term "promotion of complex formation" may refer to promotion of 10S complex formation or promotion of 7S complex formation, and may also refer to promotion of 20S complex formation. In the process of these complex formations, m-tomosyn is the protein having the highest affinity to syntaxin among the proteins that bind to syntaxin, and therefore plays a central role in the exocytosis of neurotransmitters. It is considered that the polypeptide and peptide of the present invention have an action that promotes the formation of these complexes similarly to m-tomosyn.

High level expression of m-tomosyn in PC 12 cells inhibits calcium-dependent exocytosis by 30% (non-patent document 1). Exocytosis is also inhibited in yeast strains with a deleted homolog of m-tomosyn, which results in inhibition of proliferation (non-patent document 4). These facts show that m-tomosyn plays a regulating role in this step. In other words, overexpressed tomosyn generates an inhibitory effect on 10S complex formation. Further, when tomosyn is not present, the step does not progress, which results in down-regulation of exocytosis as in the case where tomosyn is overexpressed. It is considered that the polypeptide and peptide of the present invention have a function to regulate this step by binding to syntaxin in a similar manner to m-tomosyn.

It is disclosed and reported in the aforementioned database that KIAA1006 is specifically expressed in the brain. Therefore, it is believed that the KIAA1006 polypeptide is an important factor in humans, as m-tomosyn in rats, to regulate the docking and fusion of synaptic vesicles through syntaxin.

The polypeptide of the present invention can be obtained by known genetic engineering techniques based on the nucleotide sequence information of KIAA1006 cDNA (GenBank accession number: AB023223). For example, the polypeptide can be obtained by amplifying the gene encoding the ORF region of KIAA1006 gene by the polymerase chain reaction (PCR) using a plasmid hk10084 that contains KIAA1006 which was purchased from Kazusa DNA Research Institute, as a template, and then infecting an insect cell, Sf9, with a baculovirus integrated with the gene to induce expression, as described above or in the hereinafter-described example. Further, it is possible to obtain the polypeptide of the present invention by, for example, amplifying the gene of interest using a human brain derived cDNA library as a template and a suitable primer designed by a known method based on the aforementioned KIAA1006 cDNA nucleotide sequence information, and expressing the resultant gene by a known genetic engineering technique.

As used herein, the term "polypeptide" refers to a long chain peptide such as a protein containing two or more amino acids that are bound to one another by a peptide bond or a modified peptide bond. A short chain peptide, which is also called an "oligopeptide" or "oligomer", is referred to as only a "peptide".

The polypeptide of the present invention is a polypeptide having a function to bind to human syntaxin-1a (syntaxin-1a) and comprises the amino acid sequence shown in SEQ ID NO: 1 in the Sequence Listing.

The polypeptide of the present invention may be a polypeptide that binds to human syntaxin-1a (syntaxin-1a) and contains a polypeptide comprising the amino acid sequence shown in SEQ ID NO: 1 in the Sequence Listing.

The polypeptide of the present invention may also be a polypeptide that binds to human syntaxin-1a (syntaxin-1a) and has a homology of 90% or more to the polypeptide shown in SEQ ID NO: 1 in the Sequence Listing at an amino acid sequence level. Techniques for determining the homology of amino acid sequences are known. Examples of useful methods include a method in which an amino acid sequence is determined directly or a method in which a nucleotide sequence of cDNA is first determined and the amino acid sequence encoded thereby is then deduced. It is possible to determine the ability of binding to human syntaxin-1a (syntaxin-1a) by the method described hereinafter in the Example.

Further, based on the thus specified polypeptide, a polypeptide comprising an amino acid sequence having a mutation such as a deletion, substitution, addition or insertion of one amino acid or more, for example, 1 to 100, preferably 1 to 30, more preferably 1 to 20, further preferably 1 to 10, and particularly preferably one or several amino acids, is also provided by referring to the ability of binding to human syntaxin-1a. A polypeptide having such mutation may be a naturally existing polypeptide or a polypeptide in which a mutation is introduced. Methods for introducing a mutation are well known, for example, methods involving site-specific mutagenesis, genetic homologous recombination, primer extension, polymerase chain reaction (PCR) or the like can be used alone or in suitable combination. The above methods can be carried out, for example, according to a method shown in the publications (non-patent document 5, non-patent document 6 and non-patent document 7) or by modified methods thereof. The Ulmer's techniques (non-patent document 8) may also be utilized.

When introducing a mutation as described above, in view of avoiding a change in the fundamental properties (such as physical properties, function, physiological activity, and immunological activity) of the polypeptide, mutual substitution among homologous amino acids (polar amino acids, non-polar amino acids, hydrophobic amino acids, hydrophilic amino acids, positively-charged amino acids, negatively-charged amino acids and aromatic amino acids or the like) may be readily conceived. In addition, these available peptides can be modified to the extent that no significant functional change is involved, such as modification of their constituent amino group or carboxyl group or the like by an amidation or the like.

The peptide of the present invention includes a peptide containing a partial sequence of the polypeptide shown in SEQ ID NO: 1 in the Sequence Listing. The peptide preferably comprises 5 or more, more preferably 8 or more, further preferably 12 or more, and particularly preferably 15 or more consecutive amino acids as its minimum unit. These peptides can be produced using a known method for producing a peptide in accordance to their amino acid sequence information. Examples of known methods for chemical synthesis of a peptide include solid phase synthesis, liquid phase synthesis and the like, and any method thereof can be used herein. Alternatively, the peptide can be obtained by the same method as that for the polypeptide mentioned above using genetic engineering techniques. The peptide can be also obtained by cleaving the polypeptide of the present invention using a suitable peptidase.

A peptide obtained by these methods can be used as a reagent or a standard substance or the like. Among these peptides, a peptide that binds to syntaxin-1a is useful as a substance that inhibits the binding of the polypeptide of the present invention to syntaxin-1a, and is also useful for screening for a substance that regulates the activity of the polypeptide of the present invention. A peptide that is immunologically recognized, such as, for example, an epitope peptide, can be used for producing an antibody that is specific to the polypeptide of the present invention, as an antigen by itself or by binding it to a carrier (e.g., keyhole limpet hemocyanin or egg albumin), as described hereafter.

More preferably, the polypeptide or peptide of the present invention functions to promote the formation of a complex of proteins involved in the fusion of a synaptic vesicle with a presynaptic membrane, such as, for example, the formation of a complex comprising syntaxin-1a, SNAP-25 and synaptotagmin. The polypeptide or peptide having such a function makes it possible to regulate exocytosis that occurs through the fusion of synaptic vesicles with the presynaptic membrane, by its binding function with human syntaxin-1a. More specifically, an exocytosis regulator containing the polypeptide or peptide is included within the scope of the present invention.

The polypeptide or peptide of the present invention may be a product in a cell in which it is expressed by a genetic engineering technique, a synthesized product of a cell-free system, a chemically synthesized product, or a product prepared from the cell or any biological samples, as well as the further purified products from the foregoing. Further, the polypeptide or peptide of the present invention can be a labeled one to which another protein or peptide or the like is conjugated at the N-terminal side or C-terminal side thereof, directly or indirectly via a linker peptide or the like by use of a genetic engineering technique, as long as a function (e.g., binding to syntaxin-1a) of the polypeptide or peptide of the present invention is not inhibited. Preferably, the labeling is conducted in a manner that does not inhibit the fundamental properties of the polypeptide or peptide. Examples of the protein or peptide or the like that may be conjugated include an enzyme such as glutathione S-transferase, β-galactosidase, horseradish peroxidase (HRP) or an alkalin phosphatase; tag peptides such as His-tag, Myc-tag, HA-tag, FLAG-tag, or Xpress-tag; a fluorescent substance such as green fluorescent protein, fluorescein isothiocyanate or phycoerythrin; a maltose binding protein; Fc fragment of immunoglobulin; biotin; and a radioisotope. However, the examples are not limited thereto. When measuring the protein or peptide or the like itself used in the aforementioned labeling or a function thereof, it is possible to detect, for example, binding of the polypeptide of the present invention to syntaxin-1a, which makes it easier to detect or purify of the polypeptide of the present invention.

### (Polynucleotide)

According to one embodiment of the present invention, there is provided a polynucleotide containing the nucleotide sequence encoding the aforementioned polypeptide or peptide, or the complementary sequence thereof. For example, the polynucleotide may be a polynucleotide comprising the nucleotide sequence encoding the polypeptide comprising the amino acid sequence shown in SEQ ID NO: 1 in the Sequence Listing or the complementary sequence thereof. Herein, a polynucleotide comprising the complementary sequence of a certain polynucleotide is also referred to as a "complementary strand". These polynucleotides provide, for example, genetic information that is useful for producing the polypeptide or peptide described above, or can be used as reagents or reference standards in relation to a nucleic acid.

According to another embodiment of the present invention, there is provided a polynucleotide that hybridizes under stringent conditions to a corresponding region of a polynucleotide comprising the nucleotide sequence encoding the amino acid sequence of the aforementioned polypeptide or peptide, for example, the amino acid sequence shown in SEQ ID NO: 1 in the Sequence Listing, or the complementary sequence thereof. The hybridization conditions can be employed in accordance with, for example, the method shown in the publication (non-patent document 5) or the like. These polynucleotides can be those that hybridize to the objective polynucleotide, and need not have a complementary sequence. For example, these polynucleotides may be polynucleotides that have homology of preferably 85% or more to the nucleotide sequence of the KIAA1006 gene that encodes the KIAA1006 polypeptide, or the complementary sequence thereof, more preferably, a homology of 90% or more, and further preferably, a homology of 95% or more.

The polynucleotide of the present invention includes a polynucleotide or oligonucleotide comprising 10 or more consecutive nucleotides located in the region of the predetermined nucleotide sequence of the above-mentioned polynucleotide, preferably comprising 15 or more consecutive nucleotides, and more preferably comprising 20 or more consecutive nucleotides.

The polynucleotide of the present invention may be a polynucleotide to which a gene (including a gene encoding an enzyme such as glutathione S-transferase, β-galactosidase, horseradish peroxidase (HRP) or alkalin phosphatase; a tag-peptide such as His-tag, Myc-tag, HA-tag, FLAG-tag or Xpress-tag, and the green fluorescent protein) is ligated at the 5'-terminal side or C-terminal side thereof, as long as, for example, the expression of a polypeptide encoded by the polynucleotide or a function of the expressed polypeptide is not inhibited thereby.

These polynucleotides are useful for the production of the polypeptide or peptide or the like of the present invention. Further, these polynucleotides are also useful as probes or primers for detecting a gene encoding the polypeptide of the present invention or mRNA thereof, or as antisense oligonucleotides or the like for regulating the expression of the gene. In this respect, the polynucleotide of the present invention includes not only a translated region but also a region corresponding to a non-translated region. It is also preferable to use a nucleotide sequence of a unique region in the gene encoding the polypeptide of the present invention in order to specifically inhibit the expression of the gene by an antisense oligonucleotide.

Selection of a polynucleotide encoding the polypeptide or peptide of the present invention can be carried out, for example, by confirming the expression of the protein expressed by a known protein-expression system, using, in particular, binding to human syntaxin-1a as an index of a function thereof. For the protein-expression system, for example, a cell-free protein-expression system such as a ribosome system derived from wheat germ or rabbit reticulocyte or the like can be used (non-patent document 9).

### (Recombinant Vector)

A recombinant vector can be obtained by integrating the polynucleotide described above into an suitable vector DNA. The vector DNA to be used may be selected appropriately according to the type of host and the purpose of use. The vector DNA may be a vector extracted from a natural source, or may be a vector that lacks a part of the DNA other than that necessary for replication. Examples of the vector DNA include a vector derived from a chromosome, episome and virus (such as a vector derived from a bacteria plasmid, a bacteriophage, a transposon, a yeast episome, an inserted element, and a yeast chromosome element, for example, as well as a vector derived from a virus such as baculovirus, papovavirus, SV40, vaccinia virus, adenovirus, fowlpox virus, pseudo-rabies virus and retrovirus), as well as a vector combining the same, such as, for example, a vector derived from a genetic element of a plasmid and bacteriophage such as a cosmid or a phagemid. In addition, expression vectors, cloning vectors, and the like can be used depending on the purpose.

A recombinant vector is composed of the target gene sequence and a gene sequence that carries information relating to replication and regulation (such as a promoter, a ribosome binding region, a terminator, a signal sequence and an enhancer and the like), and is produced by combining them by using a known method. A method for integrating a polynucleotide into the vector DNA can be carried out with a known method. For example, a method may be used that comprises: selecting a suitable restriction enzyme to treat the DNA to cleave it at a specific site, mixing the cleaved DNA with a DNA to be used as a vector that has been treated in the same manner, and finally re-ligating them with a ligase. Alternatively, a desired recombinant vector can also be obtained by ligating a suitable linker to the polynucleotide, and integrated it into the multiple cloning site of a vector suitable for each purpose.

In the examples described hereinafter, the plasmid hk10084 containing KIAA1006, which was purchased from the Kazusa DNA Research Institute, was used as a template to amplify the full-length ORF of KIAA1006 and a C-terminal truncated part thereof by PCR, which were integrated into pFastBacI, respectively, to obtain the baculovirus expression plasmids pFB-1006 (full length) and pFB-1006 (C-terminal truncated). These two clones were transpositioned to DH10BAC, respectively, to obtain the bacmids Bac-1006 and Bac-1006T. Although a baculovirus vector is used in the present example, a vector for use in the present invention is not limited thereto.

### (Transformant)

A transformant can be obtained by transfecting a vector DNA, into which the above polynucleotide is integrated, into a known host by a known method. The vector DNA to be transfected into a host may be one kind of vector DNA or a combination of two or more kinds of vector DNA. Examples of a host include Escherichia coli, yeast, Bacillus subtilis, insect cells, or animal cells. Introduction of the vector DNA into a host cell may be carried out by application of a known method, for example, by a standard method shown in the publication (non-patent document 5). As preferable systems, the method of integration into chromosomes can be cited in consideration of gene stability; however as a simple method, an autonomous replication system using an extranuclear gene can be used. Specifically, examples of the method include calcium phosphate-transfection, DEAE-dextran mediated transfection, microinjection, cationic lipid-mediated transfection, electroporation, genetic transduction, scrape loading, ballistic transfection, and infection. In the example described hereinafter, an insect cell is used; however a method is not limited thereto (patent 1 and patent 2).

The polypeptides or peptides of the present invention can be provided when an expression vector is used as the vector DNA for use in transformation of the host. The transformant (into which an expression vector DNA containing the aforementioned polynucleotide has been integrated) can be cultured under culture conditions and by culture methods that are suitable for each host and well-known to one skilled in the art. Culturing can be performed by referring to the function (e.g., binding to syntaxin 1a) of the polypeptide or peptide of the present invention that is expressed by the transformant. Alternatively, culturing can be performed by referring to the quantity of polypeptides or peptides generated inside or outside the host; passage culture or batch culturing can also be performed by referring to the quantity of transformant in the culture medium.

### (Collection of the Polypeptide or Peptide)

Collection and/or purification of the polypeptide or peptide of the present invention from a culture media in which the transformant was cultured or from the transformant can be carried out by referring to the presence or absence of a function (e.g., binding to human syntaxin-1a) of the polypeptide or peptide. Examples of a method for recovery and/or purification include a fractionation method based on differences in solubility using ammonium sulfate or alcohol and the like, gel filtration, ion column chromatography, and affinity chromatography, which may be used alone or in combination. Preferably, a method can be used wherein polypeptides or peptides are specifically adsorbed and collected by using polyclonal antibodies or monoclonal antibodies that can be prepared against the polypeptides or the peptides based on the information of their amino acid sequences.

### (Antibody)

An antibody is prepared by using the polypeptide or peptide of the present invention as an antigen. An antigen may be the polypeptide or peptide, or a fragment thereof. The antigen can be composed of at least 8, or preferably at least 10, or more preferably at least 12, or further preferably 15 or more amino acids. In order to prepare an antibody that is specific to the polypeptide and/or peptide, it is preferable to use a region consisting of the amino acid sequence that is unique to the polypeptide or peptide. The amino acid sequence of this region does not necessarily need to be homologous or identical to that of the polypeptides or peptides. The amino acid sequences of the sites that are exposed to the exterior of the tertiary structure of the polypeptide or peptide are preferred. Even if the amino acid sequences at the exposed sites are discontinuous in the primary structure, it suffices as far as they are amino acid sequences that are continuous on the exposed site. The antibody is not particularly limited as long as it is an antibody that immunologically binds to or recognizes the polypeptide and/or peptide. The presence or absence of the binding or recognition can be determined by a known antigen-antibody binding reaction.

A known antibody production method can be used to produce the antibody. The antibody can be obtained, for example, administering the antigen to an animal with or without linking such to a carrier, in the presence or absence of an adjuvant, to induce immunity such as a humoral response and/or cell-mediated response. The carrier is not limited, as long as it does not exert any detrimental effects by itself on the host and is capable of enhancing the antigenicity. Examples of a carrier include cellulose, polymerized amino acids, albumin, and keyhole limpet hemocyanin. Examples of an adjuvant that can be used herein include Freund's complete adjuvant (FCA), Freund's incomplete adjuvant (FIA), Ribi (MPL), Ribi (TDM), Ribi (MPL+TDM), whooping cough vaccine (Bordetella pertussis vaccine), muramyl dipeptide (MDP), aluminum adjuvant (ALUM), and combinations thereof. Mice, rats, rabbits, goats or horses are suitable animals for immunization.

A polyclonal antibody can be obtained from the serum of the animal that was treated with the immunization means, by any suitable method for collecting an antibody. For a preferable method for collecting an antibody, immune-affinity chromatography method may be exemplified.

A monoclonal antibody can be produced by collecting antibody-producing cells (e.g., lymphocytes derived from spleen or lymph nodes) from an animal that was treated with the immunizing means, and then carrying out a transformation technique using a permanently proliferating cell (for example, a myeloma strain such as P3X63Ag8 cells.) For example, the antibody producing cell is fused with a permanently proliferating cell by a known method to form a hybridoma, and then cloning of the hybridoma is performed, followed by selecting the hybridoma that produces an antibody recognizing specifically the aforementioned polypeptides and/or peptides. The antibody can be collected from the cultured solution of the hybridoma.

The thus-obtained polyclonal antibody or monoclonal antibody that can recognize and bind to the polypeptide or peptide of the present invention can be used as an antibody for purification of the polypeptide or peptide, a reagent, a labeling marker or the like. Specifically, an antibody that inhibits the function of the polypeptide of the present invention can be used for regulating the function of the polypeptide, and is useful for elucidating, preventing, improving and/or treating many kinds of disease caused by an abnormality of the polypeptide. For example, an antibody that inhibits the binding of the polypeptide to human syntaxin-1a can be used, for example, for regulating the release of neurotransmitters, and it is therefore useful for elucidating, preventing, improving and/or treating many kinds of disease caused by the release of an abnormal amount of neurotransmitters.

### (Identification and screening of compound)

The polypeptide or peptide of the present invention, the polynucleotide of the present invention, the vector into which the polynucleotide is integrated, the transformant transfected with the vector, the protein synthesis system using these, or the antibody that recognizes immunologically the polypeptide and/or peptide provide means that are useful for identifying or screening a binding inhibitor or binding stabilizer that inhibits or stabilizes the binding of the polypeptide or peptide of the present invention to a protein that interacts with the polypeptide or peptide, or an expression inhibitor or expression promoter that inhibits or promotes the expression of the polynucleotide. The method can be carried out by use of known pharmaceutical screening systems. For example, the aforementioned means and method enable screening for antagonists by means of drug design based on the tertiary structure of the polypeptide or peptide, screening for an inhibiting agent or a promoting agent of the expression at the gene level using a protein synthesis system, or screening for a substance recognized by an antibody using the antibody, and the like.

More specifically, it is possible to identify a compound that inhibits or stabilizes the binding of the polypeptide or peptide of the present invention to a protein that interacts with the polypeptide or peptide, by introducing a system to detect the binding of the polypeptide or peptide of the present invention to a protein that interacts with the polypeptide or peptide as a signal (marker), and then detecting the presence, absence or change of the signal (marker) under conditions in which the test substance is present or absent.

For example, by referring to the binding of the polypeptide or peptide of the present invention to human syntaxin-1a, a compound can be selected that is capable of influencing the binding. The binding of the polypeptide or peptide according to the present invention to human syntaxin-1a can be detected by a binding assay system that uses a known immunological method or the like.

For example, a method used in the example described hereunder can be used. In short, the binding of the polypeptide or peptide of the present invention to syntaxin-1a can be determined quantitatively by using GST-fused syntaxin-1a obtained by use of a genetic engineering technique, binding it to glutathione-Sepharose, and determining the binding of the polypeptide or peptide of the present invention thereto using an antibody against the polypeptide or peptide according to the present invention, for example, an antibody labeled with HRP (horse radish peroxidase), alkalin phosphatase, a radioisotope, a fluorescent substance or biotin or the like. Alternatively, when using a polypeptide or peptide of the present invention to which a tag-peptide is fused, the quantitative determination can be carried out by use of the anti-tag-antibody. A polypeptide of the present invention that is directly labeled with the above-described enzyme, radioisotope, fluorescent substance, biotin or the like may also be used. A secondary antibody labeled with the above-described enzyme, radioisotope, fluorescent substance, biotin or the like may also be used.

The effect of the test substance on the binding of the polypeptide or peptide of the present invention to syntaxin-1a can be determined by comparing the amount of the polypeptide or peptide of the present invention bound to syntaxin-1a in the presence of the test substance with that in the absence of the test substance. When the amount of the polypeptide or peptide of the present invention that binds to syntaxin-1a in the presence of the test substance is less than that in the absence of the test substance, it can be determined that the test substance has an effect to inhibit the binding of the polypeptide or peptide to syntaxin-1a. In contrast, when the amount of the polypeptide or peptide of the present invention that binds to syntaxin-1a in the presence of the test substance is greater than that in the absence of the test substance, it can be determined that the test substance has an activity to stabilize the binding of the polypeptide or peptide to syntaxin-1a.

Alternatively, a two-hybrid method that is well known in the art can be used. For example, identification of a test substance can be achieved by transfecting into yeast or a eukaryotic cell or the like of a plasmid that expresses the fusion protein of the polypeptide or peptide of the present invention and a DNA-binding protein, a plasmid that expresses the fusion protein of syntaxin-1a and a transcription activation protein, and a plasmid containing a reporter gene such as lacZ with a connected suitable promoter gene, and then comparing the expression amount of the reporter gene in the presence of the test substance with that in the absence of the test substance. When the expression amount of the reporter gene in the presence of the test substance is less than that in the absence of the test substance, it can be determined that the test substance has an activity to inhibit the binding of the polypeptide or peptide to syntaxin-1a. In contrast, when the expression amount of the reporter gene in the presence of the test substance is greater than that in the absence of the test substance, it can be determined that the test substance has an activity to stabilize the binding of the polypeptide or peptide to syntaxin-1a.

It is possible to use of a surface plasmon resonance sensor, such as BIACORE system, to identify a compound that influences the binding of the polypeptide or peptide of the present invention to syntaxin-1a.

It is possible to use of a method in which a scintillation proximity assay (SPA) or a fluorescence resonance energy transfer (FRET) is applied, to identify a compound that influences the binding of the polypeptide or peptide of the present invention to syntaxin-1a.

The syntaxin-1a used in these screening systems can be a protein in which a part thereof has been deleted or to which another protein has been added, as long as it does not influence the binding to the polypeptide or peptide of the present invention.

It is possible to identify a compound that inhibits or stabilizes the expression of the polynucleotide of the present invention, by selecting conditions that allows the expression of the polynucleotide, contacting the polynucleotide with a test compound under those conditions, introducing a system that uses a signal capable of detecting the presence or the absence of the expression, and detecting the presence or absence or change of the signal. As a signal to be used herein, tag-proteins such as glutathione S-transferase, His-tag, Myc-tag, HA-tag, FLAG-tag or Xpress-tag and green fluorescent protein or the like are available.

Alternatively, a compound that influences the expression of a gene of the present invention can be identified by, for example, preparing a vector in which a reporter gene, in place of the gene encoding the peptide of the present invention, is connected downstream of the promoter region of the gene, then contacting the test substance with a cell (such as an eukaryotic cell) into which the vector is transfected, and finally detecting the presence, absence or change of expression of the reporter gene. A gene that is generally used in a reporter assay can be used as the reporter gene; for example, a gene having enzyme activity such as luciferase, β-galactosidase or chloramphenicol acetyl transferase can be used. Detection of the expression of the reporter gene can be carried out by detecting the activity of the gene product. In the case of using the gene exemplified above, detection of the expression of the reporter gene can be carried out by detecting the enzyme activity.

### (Compound)

A compound identified as described above is available as a candidate compound for a binding inhibitor, binding antagonist, or binding stabilizer relating to a polypeptide of the present invention. Those compounds are also available as candidate compounds for an expression inhibitor or expression stimulator for the aforementioned polypeptide at a gene level. It can be expected that these candidate compounds will have an effect of preventing and/or treating many kinds of diseases caused by an increase, decrease or lack of expression or activity of the polypeptide of the present invention. For example, a candidate compound can be used for regulating the release of a neurotransmitter, so that it can be expected to have an effect of preventing and/or treating diseases caused by abnormal release of neurotransmitters and an abnormal amount of released neurotransmitters.

### (Pharmaceutical Composition)

The thus-selected candidate compounds can be used to prepare pharmaceutical compositions by further selecting, with consideration given to the balance between the biological effectiveness and toxicity thereof. The polypeptide or peptide of the present invention, the polynucleotide of the present invention, the plasmid of the present invention, and the antibody that immunologically recognizes the polypeptide or peptide can be used themselves as a diagnosis means (such as a diagnosis marker or reagent), as well as a pharmaceutical means such as therapeutics (e.g., an inhibitor, an antagonist, or stimulator) that utilizes the function of inhibiting, antagonizing, or promoting the activity and/or expression of the polypeptide according to the present invention. More specifically, the present invention provides a pharmaceutical composition containing at least one of the above substances, by utilizing one of them or by utilizing a combination of them.

When the expression of the polypeptide of the present invention and the activity thereof are excessive, an effective amount of the aforementioned inhibitor can be administrated with a pharmaceutically acceptable carrier to inhibit the activity of the polypeptide and thereby improve the abnormal symptoms. Further, it is possible to inhibit the expression of the endogenous gene that encodes the polypeptide by using an expression blocking method. The expression of the gene can be inhibited by using an antisense sequence of the gene that is made to be produced in a cell or is particularly administrated. These oligonucleotides can be designed and synthesized on the basis of the polynucleotide of the present invention. The oligonucleotide itself can be administrated, or related oligomers can be expressed in vivo.

For treatment of an abnormal symptom relating to a decrease or lack of expression of the polypeptide of the present invention and activity thereof, an effective amount of the agent for promoting the activities of the gene encoding the polypeptide or peptide can be administrated with a pharmaceutically acceptable carrier and thereby improve the abnormal symptom. Alternatively, it is possible to produce the polypeptide in the cell of the target by use of gene therapy. Gene therapy using the polynucleotide of the present invention can be performed according to a known method. For example, a replication-deficient retrovirus vector into which the polynucleotide of the present invention has been integrated may be prepared and used in gene therapy. It is also possible in gene therapy to produce a protein used in the therapy in the target itself. For example, using DNA or RNA that encodes the protein, a cell derived from the target can be treated ex vivo by use of, e.g., a retrovirus plasmid vector, and then the cell can then be introduced into the target.

The polypeptide of the present invention binds to syntaxin-1a, and thus, it is considered that in particular, the polypeptide relates to a disease caused by an abnormal release of neurotransmitters or an abnormal amount thereof. Therefore, the inhibitor or antagonist mentioned above is useful for preventing and/or treating the disease. A method for promoting or inhibiting the release of neurotransmitters, wherein the method comprises inhibiting or stabilizing binding of a polypeptide of the present invention to syntaxin-1a, and a method for preventing and/or treating a disease caused by an abnormal amount of neurotransmitters are included within the scope of the present invention.

In terms of a formulation, the pharmaceutical composition of the present invention is preferably formulated in combination with a suitable pharmaceutical carrier. Such a formulation comprises a therapeutically effective amount of at least one member selected from the group consisting of the polypeptide or peptide, the polynucleotide, the vector, the transformant, the antibody and the compound of the present invention, and a pharmaceutically acceptable carrier or vehicle. Examples of a carrier include physiological saline, buffered physiological saline, dextrose, water, glycerol, ethanol, and a mixture thereof; however, it is not limited thereto. The formulation may be selected according to an administration route, and such formulations are well-known to those skilled in the art. At the time of formulation, the compositions may be used alone or in combination with other compounds or medicine required for treatment.

In terms of the mode of administration, it may be either systemic administration or local administration. One preferred mode of systemic administration is injection, e.g., an intravenous injection. Other injection routes, such as subcutaneous, intramuscular, or intraperitoneal injection, may also be used. Another mode of administration may be oral administration, so long as an enteric formulation or a capsule formulation can be suitably formulated. In addition, transmucosal administration or percutaneous administration, which comprises using a penetrant such as bile salt, fusidic acid, or other surfactants, may also be used. In a local administration, forms such as ointment, paste, or gel, may be used.

Suitable dosage ranges can be determined according to the following: effectiveness of a therapeutically effective amount of at least one member selected from the group consisting of the polypeptide or peptide, the polynucleotide, the vector, the transformant, the antibody and the compound of the present invention; the route of administration; the properties of the formulation; the symptomatic conditions of the subject; and the judgment of the doctor in charge. More specifically, a suitable dosage may fall within the range of, e.g., 0.1 µg to 10 µg per 1 kg of the body weight of the subject. However, the dosage may be altered using common conventional experiments for optimization of a dosage that are well known in the art.

Preparation of a pharmaceutical may be carried out by well-known formulating procedures in accordance with the physical properties of the substance employed, for example, peptides, proteins, oligonucleotides, antibodies, or compounds. Specifically, formulations such as powdered drugs, pills, tablets, capsules, aqueous solutions, liposomal formulations, fat emulsions, clathrates (such as those of cyclodextrin), and the like can be used.

Powdered drugs, pills, capsules, or tablets can be prepared using, for example, an excipient such as lactose, glucose, sucrose, or mannitol; a disintegrant such as starch or sodium arginate; a lubricant such as magnesium stearate or talc; a binder such as polyvinylalcohol, hydroxypropyl cellulose, or gelatin; a surfactant such as fatty acid ester; and a plasticizer such as glycerin, or the like. For preparation of a tablet or capsule, a solid pharmaceutical carrier is used.

A suspension can be prepared using water; saccharides such as sucrose, sorbitol, or fructose; glycols such as PEG; and oils.

Injectable solutions can be prepared using a carrier comprising a saline solution, a glucose solution, or a mixture of the salt water and glucose solution.

Inclusion into a liposome formulation may be conducted in the following manner: by dissolving the substance of interest in a solvent (e.g., ethanol) to make a solution, adding a solution of phospholipids dissolved in an organic solvent (e.g., chloroform), removing the solvent by evaporation and adding a phosphate buffer thereto, agitating the solution and then subjecting it to sonication followed by centrifugation to obtain supernatant, and finally, filtrating the supernatant for recovering a liposome.

Fat emulsion can be prepared in the following manner: by mixing the substance of interest, an oil ingredient (vegetable oil such as soybean oil, sesame oil, olive oil, or MCT), an emulsifier (such as phospholipid), and the like; heating the mixture to make a solution; adding water of a required quantity; and then emulsifying or homogenizing by use of an emulsifier (homogenizer, e.g., a high pressure jet type, an ultrasonic type, or the like). The fat emulsion may be also lyophilized. For conducting lipid-emulsification, an auxiliary emulsifier may be added, and examples thereof include glycerin or saccharides (e.g., glucose, sorbitol, fructose, etc.).

Inclusion into a cyclodextrin formulation may be carried out in the following manner: by dissolving the substance of interest in a solvent (e.g., ethanol); adding a solution of cyclodextrin dissolved in water under heating thereto; chilling the solution and filtering the precipitates; and drying under sterilization. At this time, the cyclodextrin to be used may be appropriately selected from among those having different void sizes (α, β, or γ type) in accordance with the bulkiness of the substance.

### (Measurement Method and Reagent for Diagnosis)

The polypeptide or peptide of the present invention, polynucleotide of the present invention, or the antibody that immunologically recognizes the polypeptide or peptide can be used itself as a marker for diagnosis or a reagent. When used as reagents, they may contain a substance such as a buffer, salt, stabilizer, and/or antiseptic agent. The present invention also provides a reagent kit comprising one or more containers in which one or more of the substances mentioned above are filled. In terms of the preparation thereof, it is sufficient to use a well-known means for the preparation suitable for a polypeptide or peptide, protein, polynucleotide, or an antibody, respectively.

These reagents and reagent kits can be used in the identification method of the present invention. The reagents and reagent kits can also be used for quantitative and/or qualitative measurement of the polypeptide or peptide of the present invention, or a polynucleotide encoding either one thereof. A method for performing the measurement can be constructed using a method that is known to those skilled in the art. Examples of methods that can be used in the measurement of the polypeptide or peptide include radioimmunoassay, competitive binding assay, Western blotting assay and ELISA assay. The polynucleotide can be detected and quantitatively determined at the nucleic acid level, for example, the RNA level, by use of, for example, amplification, PCR, RT-PCR, RNase protection, Northern blotting, and other methods of hybridization.

Further, the test or diagnosis of a disease caused by the expression or activity of the polypeptide is enabled by qualitatively or quantitatively measuring the polypeptide or peptide of the present invention or a nucleic acid encoding one of these as a diagnosis marker. A method for the test or the diagnosis can be performed, for example, by using the interaction or reactivity with the polynucleotide encoding the aforementioned polypeptide or peptide to detect the presence of the corresponding nucleic acid, to determine the existing amount thereof, and/or to identify a mutation thereof, as well as by determining the in vivo distribution of the polypeptide or peptide in an individual, by detecting the presence of the polypeptide or peptide, by determining the existing amount thereof, and/or by detecting a mutation thereof.

Examples of a sample include a sample derived from an individual such as a cell, blood, urine, saliva, spinal fluid, tissue biopsy or autopsy specimen. A nucleic acid can be obtained from each sample mentioned above by a known method. A nucleic acid can be used directly for detection in the form of genome DNA, or may be used after being enzymatically amplified by PCR or other amplifying methods before the analysis. RNA or cDNA may also be used in the same way. A deletion or insertion can be detected by a change in size of an amplified product when compared to the size of the normal gene type. A point mutation can be identified by hybridization of the amplified DNA to a labeled DNA encoding the polypeptide of the present invention.

Detecting a mutation, decrease or increase in the polypeptide of the present invention and a nucleic acid encoding the polypeptide, enables diagnosis of a disease caused by the abnormal release of neurotransmitters or an abnormal amount thereof.

### Examples

Hereinafter, the present invention may be explained more particularly with an example; however, the present invention is not limited to the following example.

### (Isolation of Genes)

A homology search was performed for a clone having homology to the VAMP homologous region of rat m-tomosyn using the human brain-derived long chain cDNA library database of the Kazusa DNA Research Institute. As a result, it was found that KIAA1006 has homology to rat m-tomosyn of about 57.6% at the nucleotide level and of about 67.9% at the amino acid level.

In the database of the Kazusa DNA Research Institute, it was pointed out that KIAA1006 may be a partial sequence with a N-terminal truncation. Therefore, in order to determine the presence of a translation initiation site in the upstream region of KIAA1006 gene, 5'-rapid amplification of cDNA ends (RACE) was carried out using human polyA(+) RNA (Clontech Laboratories, Inc.), 1006 race primer (SEQ ID NO: 2 in the Sequence Listing) and SMART RACE cDNA Amplification Kit (Clontech Laboratories, Inc.).

1006 race: GCCAGGGGAGGAGGCAGTTAAGCCATC (SEQ ID NO: 2 in the
Sequence Listing).

As a result, no novel upstream sequences were found. Further, in consideration of the fact that the amino acid sequence following the first methionine within the amino acid sequence deduced from the nucleotide sequence of KIAA 1006 showed a remarkably high homology to the N-terminal sequence of rat m-tomosyn at amino acid 1-15, it was determined that the first methionine of KIAA1006 is the translation initiation site of the gene containing KIAA1006, and the following analysis was done.

### (Construction of expression plasmids for full-length ORF of KIAA1006 and C-terminal-truncated ORF of KIAA1006).

Plasmid hk10084 containing KIAA1006 was purchased from the Kazusa DNA Research Institute. Plasmid hk10084 is a plasmid produced by inserting KIAA1006 into Sal I-Not I site of pBluescript II SK(+) vector. Specifically, Plasmid hk10084 is a plasmid produced by ligating Sal I adaptor to the 5' terminal of KIAA1006 cDNA to insert the ligated DNA into the Sal I site of pBluescript II SK(+) vector, and inserting the 3' terminal of KIAA1006 cDNA into the Not I site of the vector. Plasmid hk10084 was used as a template. The ORF region of KIAA1006 was amplified using the combination of 1006 (s) (SEQ ID NO: 3 in the Sequence Listing, including the Hisx6-Tag sequence) and 1006 (a)1 (SEQ ID NO: 4 in the Sequence Listing, for the full length ORF), or the combination of 1006 (s) (SEQ ID NO: 3 in the Sequence Listing) and 1006 (a)2 (SEQ ID NO: 5 in the Sequence Listing) (for the C-terminal truncated product), and Advantage-HF2 PCR Kit (Clontech Laboratories, Inc.). The two PCR products thus obtained were digested with EcoRI and XhoI and inserted into pFastBacI (Gibco BRL Inc.) that had been treated with the same enzymes, to obtain baculovirus expression plasmids, pFB-1006 (encoding full length amino acid 1-1186) and pFB-1006t (encoding amino acid 1-1157, in which the inferred VAMP homology region was deleted from the full length amino acid). The respective bacmids, Bac-1006 and Bac-1006t, were obtained by transposition of these plasmids into competent E. Coli DH10BAC (Gibco BRL Inc.).

1006(s):CCGGAATTCGCCACCATGCATCACCATCACCATCACAAGAAG
AAGTTTAATTTCCGAAAAGTT (SEQ ID NO: 3 in the Sequence Listing)

1006(a)1:CCGCTCGAGTCAGAATTGGTACCATTTCTTATCCTTG (SEQ ID
NO: 4 in the Sequence Listing)

1006(a)2:CCGCTCGAGTCAAGTTTTCTCTTCCAGCTCTCCTAG (SEQ ID
NO: 5 in the Sequence Listing)

As used herein, "(s)" denotes a sense primer and "(a)" denotes an antisense primer.

### (Expression and Purification of KIAA1006 Polypeptide and KIAA1006 C-terminal Truncated Polypeptide in sf9 Cell).

Bac-1006 and Bac-1006t were transfected into sf9 cells (35 mm dish), respectively, by use of CellFECTIN (Gibco BRL Inc.). The culture supernatant after 3 days from the transfection was used for re-infection of the sf9 cells to amplify the baculovirus. sf9 cells were subjected to infection with the amplified virus, and 3 days later, the cells were dissolved in EM buffer (50 mM sodium phosphate/300 mM NaCl/ 1% Nonidet P-40/1 mM phenylmethanesulfonyl fluoride (PMSF)), and subjected to ultrasonic disintegration. Histidine-tagged KIAA1006 polypeptide and histidine-tagged KIAA1006 C-terminal truncated polypeptide were purified from the extraction of the obtained disintegrated cell using a cobalt resin (TALON, Clontech Laboratories, Inc.). By Western blotting using an anti-His antibody (Qiagen Inc.), it was confirmed that the histidine-tagged KIAA1006 polypeptide and histidine-tagged KIAA1006 C-terminal truncated polypeptide were recovered in almost the same amount.

It has been reported that rat m-tomosyn was expressed in sf9 cells and COS7 cells because rat m-tomosyn could not be expressed by a common method using E. coli (non-patent document 1). However, it has been also reported that even by this method, the protein obtained was insoluble, so that a protein that was renatured after being subjected to SDS-PAGE was used for analysis. The two forms of the polypeptide of the present invention that were found by the inventors could be purified as soluble proteins and could be purified using a cobalt resin, which made it possible to conduct an in-vitro binding test. The application of the present methods of expression and purification makes it possible to measure the binding/dissociation constant or the like between human syntaxin-1a and a polypeptide of the present invention, which was impossible for rat m-tomosyn by Fujita et al. (non-patent document 1).

### (Expression and Purification of Human Syntaxin-1a in E. coli)

To obtain human syntaxin-1a employing cDNA derived from human brain polyA RNA (Clontech Laboratories, Inc.) as a template, 1-266AA within the ORF region of syntaxin-1a was amplified by PCR using the two primers, syla4T(s) (SEQ ID NO: 6 in the Sequence Listing) and sy1a4T(a) (SEQ ID NO: 7 in the Sequence Listing) described below. The amplified products were digested with EcoRI and XhoI, and the digested products were then integrated into a plasmid for expression of the GST-fusion protein, pGEX-4T-3 (Amersham Pharmacia Biotech), that had been digested with EcoRI and XhoI in a similar manner, to obtain pGEX-sy1a. The sequence of pGEX-sy1a was determined, and the absence of mutagenesis or frame-shift caused by PCR was confirmed.

sy1a4T(s):CCGGAATTCCATGAAGGACCGAACCCAGGAGCTC (SEQ ID
NO: 6 in the Sequence Listing)

syla4T(a):CCGCTCGAGTTACGCCTTGCTCTGGTACTT (SEQ ID NO: 7 in
the Sequence Listing)

DH5a was transformed with pGEX-sy1a and cultured at 37°C until it entered the late proliferation phase (OD 0.5 or more). Isopropyl β-D-thiogalactopyranoside (IPTG) was added thereto at the final concentration of 1 mM, and 5 hours of expression induction was carried out. The collected bacteria was washed with PBS(-), suspended in Buffer A (50 mM tris HCl pH 7.5/1 mM EDTA/1 mM DTT/1 mM PMSF), and then treated for 30 min with lysozyme (0.5 mg/ml) in ice water. After adding Nonidet P-40 (final concentration of 1%), the bacteria was sonicated, and GST-syntaxin-1a (1-266AA) was purified by the use of glutathione-Sepharose according to a common method from the supernatant obtained after centrifugation. Expression induction was carried out in a similar manner for pGEX-4T-3 having no insertion to express and purify GST.

Syntaxin-1a that was expressed herein was not the full length of the ORF, but a protein consisting of 1-266 amino acids corresponding to syntaxin-1a with C-terminal truncation. Syntaxin-1a with C-terminal truncation consisting of 1-266 amino acids was used, since it was confirmed in the reports (non-patent document 10, non-patent document 11, non-patent document 12, and non-patent document 13) that the extremely low hydrophilicity of the 21 amino acid residues in the C-terminal side of syntaxin-1a were involved in a problem of insolubilization, and C-terminal truncation did not reduce the properties of syntaxin-1a as a protein.

As a result, a purified standard product of GST-syntaxin-1a fused protein, which became almost a single band by Coomassie brilliant blue staining, was obtained.

### (Analysis of Binding of KIAA1006 Polypeptide with Syntaxin-1a)

KIAA1006 polypeptide (the histidine-tagged KIAA1006 polypeptide described above) and its C-terminal truncated product (the histidine-tagged C-terminal truncation product of KIAA1006 polypeptide described above) purified with a cobalt resin were respectively mixed with glutathione-Sepharose (bed volume 10 µl) to which GST (17 µg) was bound, stirred for 6 hours at 4°C, and then centrifuged. The obtained supernatants (GST-glutathione-Sepharose-unadsorbed fractions) were respectively added to glutathione-Sepharose (bed volume 10 µl) to which GST-syntaxin-1a (17µg) was bound, and after stirring for 16 hours at 4°C, the resultants were subjected to centrifugation. The fractions adsorbed to GST-glutathione-Sepharose and the fractions adsorbed to GST-syntaxin-1a-glutathione-Sepharose were eluted with SDS-PAGE sample buffer, respectively, and the binding of syntaxin-1a to KIAA1006 polypeptide or to its C-terminal truncation product was analyzed by Western blotting using anti-penta His antibody (Qiagen).

For the SDS-PAGE, 4-12% Bis-Tris Gel (NOVEX) was used. The detection of a secondary antibody that was labeled with horseradish peroxidase was carried out using ECL Western blotting detection reagents (Amersham Pharmacia Biotech).

As a result, as shown in Fig. 2, in the GST-glutathione-Sepharose-adsorbed fractions, neither KIAA1006 polypeptide nor the C-terminal truncated product was detected (Fig. 2: lanes 1 and 2). In contrast, in the elutions from glutathione-Sepharose bound with GST-syntaxin-1a, a band that was detectable by anti-penta-His antibody was detected when KIAA1006 polypeptide was applied, but the C-terminal truncated product was not detected in the elution when the C-terminal truncated product was applied (Fig. 2: lanes 4 and 6).

Thus, it was revealed that the full length ORF of KIAA1006 binds to syntaxin-1a and that the C-terminal truncation product does not bind to syntaxin-1a. From these results, it was clarified that KIAA1006 polypeptide binds to syntaxin through the VAMP homologous region thereof.

### INDUSTRIAL APPLICABILITY

As described above, it was found that the clone KIAA1006, which was selected from a human brain-derived long chain cDNA library disclosed by the Kazusa DNA Research Institute, encodes a protein that binds to human syntaxin-1a. This gene has homology to rat m-tomosyn of about 57.6% at the nucleotide level and of about 67.9% at the amino acid level. It has been reported on a database that KIAA1006 is specifically expressed in the brain. It is known that syntaxin-1a is an important protein for releasing neurotransmitters, which suggest that KIAA1006 polypeptide, which is capable of binding to syntaxin-1a, also plays an important role in the release of neurotransmitters. Thus, it has been found that KIAA1006 according to the present invention, a polypeptide derived therefrom, and polynucleotides encoding these are useful for a preventive and/or therapeutic medicine for a disease relating to the polypeptide of the present invention, for example, a disease caused by abnormal release of neurotransmitters or an abnormal amount thereof, as well as for a diagnosis means.

### Sequence Listing free text.

SEQ ID NO: 1 in the Sequence Listing: an amino acid sequence of polypeptide consisting of full length KIAA1006 ORF, deduced from its cDNA sequence.
SEQ ID NO: 2 in the Sequence Listing: a primer used for analysing the upstream of N-terminus of KIAA1006 by the 5'-race method.
SEQ ID NO: 3 in the Sequence Listing: a sense primer used for amplifying the partial sequence of KIAA1006 ORF by PCR.
SEQ ID NO: 4 in the Sequence Listing: an antisense primer used for amplifying the partial sequence of KIAA1006 ORF by PCR.
SEQ ID NO: 5 in the Sequence Listing: an antisense primer used for amplifying the partial sequence of a C-terminal deletion mutant of KIAA1006 ORF by PCR.
SEQ ID NO: 6 in the Sequence Listing: a sense primer used for amplifying the sequence of syntaxin 1 a by PCR.
SEQ ID NO: 7 in the Sequence Listing: an antisense primer used for amplifying the sequence of syntaxin-1a by PCR.

## Claims

1. A polypeptide having a function of binding to human syntaxin-1a, wherein the polypeptide is selected from the group consisting of:
(i) a polypeptide comprising the amino acid sequence shown in SEQ ID NO: 1 in the Sequence Listing;
(ii) a polypeptide containing the polypeptide of (i);
(iii) a polypeptide having at least 90% homology to the amino acid sequence of the polypeptide of (i); and
(iv) a polypeptide having a mutation such as a deletion, substitution, addition or insertion of one or several amino acids relative to the amino acid sequence of any of the polypeptides of (i) to (iii).

2. A peptide comprising at least five consecutive amino acid residues in the amino acid sequence shown in SEQ ID NO: 1 in the Sequence Listing.

3. The polypeptide according to claim 1 or the peptide according to claim 2, wherein the polypeptide or the peptide promotes the formation of a complex that comprises human syntaxin-1a, synaptosomal-associated 25kDa protein and synaptotagmin and is involved in fusion of a synaptic vesicle with a presynaptic membrane.

4. A regulator of exocytosis through fusion of a synaptic vesicle with a presynaptic membrane, wherein the regulator comprises the polypeptide according to claim 1 or the peptide according to claim 2 and has a binding function to human syntaxin-1a.

5. A polynucleotide comprising a nucleotide sequence that encodes the polypeptide according to claim 1 or the peptide according to claim 2, or the complementary sequences thereof.

6. A polynucleotide that hybridizes to the polynucleotide according to claim 5 under stringent conditions.

7. A polynucleotide comprising at least 15 consecutive nucleotides of the polynucleotide according to claim 5.

8. A recombinant vector containing the polynucleotide according to any one of claim 5 to claim 7.

9. The recombinant vector according to claim 8, wherein the recombinant vector is a recombinant expression vector.

10. A transformant that is transformed with the recombinant vector according to claim 8 or 9.

11. A method for producing the polypeptide according to claim 1 or the peptide according to claim 2, wherein the method comprises a step of incubating a transformant transformed with the recombinant vector according to claim 9.

12. An antibody that immunologically recognizes the polypeptide according to claim 1 or the peptide according to claim 2.

13. The antibody according to claim 12, which inhibits the binding of the polypeptide according to claim 1 or the peptide according to claim 2 to a protein that interacts with the polypeptide or the peptide.

14. The antibody according to claim 13, which inhibits the binding of the polypeptide according to claim 1 or the peptide according to claim 2 to human syntaxin-1a.

15. A method for identifying a compound that inhibits or stabilizes the binding of the polypeptide according to claim 1 or the peptide according to claim 2 to a protein that interacts with said polypeptide or said peptide and/or a compound that inhibits or promotes expression of the polynucleotide according to any one of claims 5 to 7, wherein the method uses at least one member selected from the group consisting of the polypeptide according to claim 1 or the peptide according to claim 2, the polynucleotide according to any one of claims 5 to 7, the vector according to claim 8 or 9, the transformant according to claim 10, and the antibody according to any one of claims 12 to 14.

16. A method for identifying a compound that inhibits or stabilizes the binding of the polypeptide according to claim 1 or the peptide according to claim 2 to a protein that interacts with the polypeptide or the peptide, wherein the method comprises steps of: contacting a test substance with the polypeptide according to claim 1 or the peptide according to claim 2, and the protein that interacts with the polypeptide or the peptide, under conditions that allow the binding of the polypeptide according to claim 1 or the peptide according to claim 2 to the protein that interacts with the polypeptide or peptide; and determining whether the test substance inhibits or stabilizes the binding of the polypeptide according to claim 1 or the peptide according to claim 2 to a protein that interacts with the polypeptide or the peptide, by detecting the presence, absence, or change of a signal generated by the binding of the polypeptide according to claim 1 or the peptide according to claim 2 to the protein that interacts with the polypeptide or the peptide.

17. A method for identifying a compound that inhibits or stabilizes the binding of the polypeptide according to claim 1 or the peptide according to claim 2 to human syntaxin-1a, wherein the method comprises steps of: contacting a test substance with the polypeptide or the peptide and human syntaxin-1a, under conditions that allow the binding of the polypeptide or the peptide to human syntaxin-1a; and determining whether the test substance inhibits or stabilizes the binding of the polypeptide or the peptide to human syntaxin-1a, by detecting the presence, absence, or change of a signal generated by the binding of the polypeptide or the peptide to human syntaxin-1a.

18. A method for identifying a compound that inhibits or promotes expression of the polynucleotide according to any one of claims 5 to 7, wherein the method comprises steps of: contacting a test substance with the polynucleotide under conditions that allow expression of the polynucleotide; and determining whether the test substance inhibits or promotes expression of the polynucleotide by detecting the presence, absence, or change of a signal generated by expression of the polynucleotide.

19. A compound that is identified by the method according to any one of claim 15 to 18.

20. A compound that inhibits or stabilizes the binding of the polypeptide according to claim 1 or the peptide according to claim 2 to human syntaxin-1a by interacting with the polypeptide or the peptide.

21. A compound that inhibits or promotes expression of the polynucleotide according to any one of claims 5 to 7 by interacting with the polynucleotide.

22. A pharmaceutical composition comprising at least one member selected from the group consisting of the polypeptide according to claim 1 or the peptide according to claim 2, the polynucleotide according to any one of claims 5 to 7, the vector according to claim 8 or 9, the transformant according to claim 10, the antibody according to claim 13 or 14, and the compound according to any one of claims 19 to 21.

23. The pharmaceutical composition according to claim 22 that has a function to promote or inhibit release of neurotransmitters.

24. The pharmaceutical composition according to claim 22, which is used as a preventive and/or therapeutic agent for a disease caused by an abnormal amount of neurotransmitters.

25. A method for promoting or inhibiting release of neurotransmitters, wherein the method comprises inhibiting or stabilizing the binding of the polypeptide according to claim 1 to human syntaxin-1a.

26. A method for preventing or treating a disease caused by an abnormal amount of neurotransmitters, wherein the method comprises inhibiting or stabilizing the binding of the polypeptide according to claim 1 to human syntaxin-1a.

27. A method for quantitatively or qualitatively measuring a polypeptide according to claim 1 or a polynucleotide encoding the polypeptide.

28. A method for measurement used in diagnosis of a disease caused by an abnormality in the expression or function of the polypeptide according to claim 1, wherein the method comprises conducting an analysis employing (A) a nucleic acid encoding the polypeptide and/or (B) the polypeptide as a marker.

29. A method for measurement used in diagnosis of a disease associated with an abnormal function of neurotransmittion caused by an abnormal amount of the polypeptide according to claim 1, wherein the method comprises conducting an analysis employing (A) a nucleic acid encoding the polypeptide and/or (B) the polypeptide as a marker.

30. A reagent kit comprising at least one member selected from the group consisting of the polypeptide according to claim 1 or the peptide according to claim 2, the polynucleotide according to any one of claims 6 to 8, and the antibody according to any one of claims 13 to 15.

31. The reagent kit according to claim 30, which is used in the method for identifying a compound according to any one of claims 15 to 18, or in the measurement method according to any one of claims 27 to 29.


SEQUENCE LISTING

<110> DAIICHI PHARMACEUTICAL CO., LTD.

<120> POLYPEPTIDE BINDING TO HUMAN SYNTAXIN-1a
<130> GP03-1007PCT

<150> JP P2002-75551
<151> 2002-03-19

<160> 7

<170> PatentIn version 3.1

<210> 1
<211> 1186
<212> PRT
<213> Homo sapiens

<220>
<221> misc_feature
<223> an amino acid sequence of polypeptide consisting of full length K
IAA1006 ORF, dedeuced from its cDNA sequence

<400> 1

Met Lys Lys Phe Asn Phe Arg Lys Val Leu Asp Gly Leu Thr Ala Ser
1               5                   10                  15

Ser Pro Gly Ser Gly Ser Ser Ser Gly Ser Asn Ser Gly Gly Gly Ala
            20                  25                  30

Gly Ser Gly Ser Val His Pro Ala Gly Thr Ala Gly Val Leu Arg Glu
        35                  40                  45

Glu Ile Gln Glu Thr Leu Thr Ser Glu Tyr Phe Gln Ile Cys Lys Thr
    50                  55                  60

Val Arg His Gly Phe Pro His Gln Pro Thr Ala Leu Ala Phe Asp Pro
65                  70                  75                  80

Val Gln Lys Ile Leu Ala Ile Gly Thr Arg Thr Gly Ala Ile Arg Ile

                85                  90                  95

Leu Gly Arg Pro Gly Val Asp Cys Tyr Cys Gln His Glu Ser Gly Ala
            100                 105                 110

Ala Val Leu Gln Leu Gln Phe Leu Ile Asn Glu Gly Ala Leu Val Ser
        115                 120                 125

Ala Ser Ser Asp Asp Thr Leu His Leu Trp Asn Leu Arg Gln Lys Arg
    130                 135                 140

Pro Ala Ile Leu His Ser Leu Lys Phe Asn Arg Glu Arg Ile Thr Tyr
145                 150                 155                 160

Cys His Leu Pro Phe Gln Ser Lys Trp Leu Tyr Val Gly Thr Glu Arg
                165                 170                 175

Gly Asn Thr His Ile Val Asn Ile Glu Ser Phe Ile Leu Ser Gly Tyr
            180                 185                 190

Val Ile Met Trp Asn Lys Ala Ile Glu Leu Ser Thr Lys Thr His Pro
        195                 200                 205

Gly Pro Val Val His Leu Ser Asp Ser Pro Arg Asp Glu Gly Lys Leu
    210                 215                 220

Leu Ile Gly Tyr Glu Asn Gly Thr Val Val Phe Trp Asp Leu Lys Ser
225                 230                 235                 240

Lys Arg Ala Glu Leu Arg Val Tyr Tyr Asp Glu Ala Ile His Ser Ile
                245                 250                 255

Asp Trp His His Glu Gly Lys Gln Phe Met Cys Ser His Ser Asp Gly
            260                 265                 270

Ser Leu Thr Leu Trp Asn Leu Lys Ser Pro Ser Arg Pro Phe Gln Thr

        275                 280                 285

Thr Ile Pro His Gly Lys Ser Gln Arg Glu Gly Arg Lys Ser Glu Ser
    290                 295                 300

Cys Lys Pro Ile Leu Lys Val Glu Tyr Lys Thr Cys Lys Asn Ser Glu
305                 310                 315                 320

Pro Phe Ile Ile Phe Ser Gly Gly Leu Ser Tyr Asp Lys Ala Cys Arg
                325                 330                 335

Arg Pro Ser Leu Thr Ile Met His Gly Lys Ala Ile Thr Val Leu Glu
            340                 345                 350

Met Asp His Pro Ile Val Glu Phe Leu Thr Leu Cys Glu Thr Pro Tyr
        355                 360                 365

Pro Asn Glu Phe Gln Glu Pro Tyr Ala Val Val Val Leu Leu Glu Lys
    370                 375                 380

Asp Leu Ile Val Val Asp Leu Thr Gln Ser Asn Phe Pro Ile Phe Glu
385                 390                 395                 400

Asn Pro Tyr Pro Met Asp Ile His Glu Ser Pro Val Thr Cys Thr Ala
                405                 410                 415

Tyr Phe Ala Asp Cys Pro Pro Asp Leu Ile Leu Val Leu Tyr Ser Ile
            420                 425                 430

Gly Val Lys His Lys Lys Gln Gly Tyr Ser Asn Lys Glu Trp Pro Ile
        435                 440                 445

Ser Gly Gly Ala Trp Asn Leu Gly Ala Gln Thr Tyr Pro Glu Ile Ile
    450                 455                 460

Ile Thr Gly His Ala Asp Gly Ser Ile Lys Phe Trp Asp Ala Ser Ala

465                 470                 475                 480

Ile Thr Leu Gln Met Leu Tyr Lys Leu Lys Thr Ser Lys Val Phe Glu
                485                 490                 495

Lys Gln Lys Val Gly Glu Gly Lys Gln Thr Cys Glu Ile Val Glu Glu
            500                 505                 510

Asp Pro Phe Ala Ile Gln Met Ile Tyr Trp Cys Pro Glu Ser Arg Ile
        515                 520                 525

Phe Cys Val Ser Gly Val Ser Ala Tyr Val Ile Ile Tyr Lys Phe Ser
    530                 535                 540

Arg His Glu Ile Thr Thr Glu Ile Val Ser Leu Glu Val Arg Leu Gln
545                 550                 555                 560

Tyr Asp Val Glu Asp Ile Ile Thr Pro Glu Pro Glu Thr Ser Pro Pro
                565                 570                 575

Phe Pro Asp Leu Ser Ala Gln Leu Pro Ser Ser Arg Ser Leu Ser Gly
            580                 585                 590

Ser Thr Asn Thr Val Ala Ser Glu Gly Val Thr Lys Asp Ser Ile Pro
        595                 600                 605

Cys Leu Asn Val Lys Thr Arg Pro Val Arg Met Pro Pro Gly Tyr Gln
    610                 615                 620

Ala Glu Leu Val Ile Gln Leu Val Trp Val Asp Gly Glu Pro Pro Gln
625                 630                 635                 640

Gln Ile Thr Ser Leu Ala Val Ser Ser Ala Tyr Gly Ile Val Ala Phe
                645                 650                 655

Gly Asn Cys Asn Gly Leu Ala Val Val Asp Phe Ile Gln Lys Thr Val

            660                 665                 670

Leu Leu Ser Met Gly Thr Ile Asp Leu Tyr Arg Ser Ser Asp Leu Tyr
        675                 680                 685

Gln Arg Gln Pro Arg Ser Pro Arg Lys Asn Lys Gln Phe Ile Ala Asp
    690                 695                 700

Asn Phe Cys Met Arg Gly Leu Ser Asn Phe Tyr Pro Asp Leu Thr Lys
705                 710                 715                 720

Arg Ile Arg Thr Ser Tyr Gln Ser Leu Thr Glu Leu Asn Asp Ser Pro
                725                 730                 735

Val Pro Leu Glu Leu Glu Arg Cys Lys Ser Pro Thr Ser Asp His Val
            740                 745                 750

Asn Gly His Cys Thr Ser Pro Thr Ser Gln Ser Cys Ser Ser Gly Lys
        755                 760                 765

Arg Leu Ser Ser Ala Asp Val Ser Lys Val Asn Arg Trp Gly Pro Gly
    770                 775                 780

Arg Pro Pro Phe Arg Lys Ala Gln Ser Ala Ala Cys Met Glu Ile Ser
785                 790                 795                 800

Leu Pro Val Thr Thr Glu Glu Asn Arg Glu Asn Ser Tyr Asn Arg Ser
                805                 810                 815

Arg Ser Ser Ser Ile Ser Ser Ile Asp Lys Asp Ser Lys Glu Ala Ile
            820                 825                 830

Thr Ala Leu Tyr Phe Met Asp Ser Phe Ala Arg Lys Asn Asp Ser Thr
        835                 840                 845

Ile Ser Pro Cys Leu Phe Val Gly Thr Ser Leu Gly Met Val Leu Ile

    850                 855                 860

Ile Ser Leu Asn Leu Pro Leu Ala Asp Glu Gln Arg Phe Thr Glu Pro
865                 870                 875                 880

Val Met Val Leu Pro Ser Gly Thr Phe Leu Ser Leu Lys Gly Ala Val
                885                 890                 895

Leu Thr Phe Ser Cys Met Asp Arg Met Gly Gly Leu Met Gln Pro Pro
            900                 905                 910

Tyr Glu Val Trp Arg Asp Pro Asn Asn Ile Asp Glu Asn Glu Lys Ser
        915                 920                 925

Trp Arg Arg Lys Val Val Met Asn Ser Ser Ser Ala Ser Gln Glu Ile
    930                 935                 940

Gly Asp His Gln Tyr Thr Ile Ile Cys Ser Glu Lys Gln Ala Lys Val
945                 950                 955                 960

Phe Ser Leu Pro Ser Gln Thr Cys Leu Tyr Val His Asn Ile Thr Glu
                965                 970                 975

Thr Ser Phe Ile Leu Gln Ala Asn Val Val Val Met Cys Ser Ser Ala
            980                 985                 990

Cys Leu Ala Cys Phe Cys Ala Asn Gly His Ile Met Ile Met Ser Leu
        995                 1000                1005

Pro Ser Leu Arg Pro Met Leu Asp Val Asn Tyr Leu Pro Leu Thr
    1010                1015                1020

Asp Met Arg Ile Ala Arg Thr Phe Cys Phe Thr Asn Glu Gly Gln
    1025                1030                1035

Ala Leu Tyr Leu Val Ser Pro Thr Glu Ile Gln Arg Leu Thr Tyr

1040                 1045                 1050

Ser Gln  Glu Met Cys Asp Asn  Leu Gln Asp Met Leu  Gly Asp Leu
    1055                 1060                 1065

Phe Thr  Pro Ile Glu Thr Pro  Glu Ala Gln Asn Arg  Gly Phe Leu
    1070                 1075                 1080

Lys Gly  Leu Phe Gly Gly Ser  Gly Gln Thr Phe Asp  Arg Glu Glu
    1085                 1090                 1095

Leu Phe  Gly Glu Ala Ser Ala  Gly Lys Ala Ser Arg  Ser Leu Ala
    1100                 1105                 1110

Gln His  Ile Pro Gly Pro Gly  Ser Ile Glu Gly Met  Lys Gly Ala
    1115                 1120                 1125

Ala Gly  Gly Val Met Gly Glu  Leu Thr Arg Ala Arg  Ile Ala Leu
    1130                 1135                 1140

Asp Glu  Arg Gly Gln Arg Leu  Gly Glu Leu Glu Glu  Lys Thr Ala
    1145                 1150                 1155

Gly Met  Met Thr Ser Ala Glu  Ala Phe Ser Lys His  Ala His Glu
    1160                 1165                 1170

Leu Met  Leu Lys Tyr Lys Asp  Lys Lys Trp Tyr Gln  Phe
    1175                 1180                 1185

<210>  2
<211>  27
<212>  DNA
<213>  Artificial

<220>
<223>  a primer used for analysing the upstream of N-terminus of KIAA100
       6 by 5'-race method

<400> 2
gccaggggag gaggcagtta agccatc 27

<210> 3
<211> 63
<212> DNA
<213> Artificial

<220>
<223> a sense primer used for amplifying the partial sequence of KIAA10
06 ORF by PCR

<400> 3
ccggaattcg ccaccatgca tcaccatcac catcacaaga agaagtttaa tttccgaaaa 60

gtt 63

<210> 4
<211> 37
<212> DNA
<213> Artificial

<220>
<223> an antisense primer used for amplifying the partial sequence of K
IAA1006 ORF by PCR

<400> 4
ccgctcgagt cagaattggt accatttctt atccttg 37

<210> 5
<211> 36
<212> DNA
<213> Artificial

<220>
<223> an antisense primer used for amplifying byPCR of the partial sequ
ence of C-terminal deletion mutant of KIAA1006 ORF

<400> 5
ccgctcgagt caagttttct cttccagctc tcctag 36

<210> 6
<211> 34
<212> DNA

<213>  Artificial

<220>
<223>  a sense primer used for amplifying the sequence of syntaxin 1a by
       PCR

<400>  6
ccggaattcc atgaaggacc gaacccagga gctc                                34


<210>  7
<211>  30
<212>  DNA
<213>  Artificial

<220>
<223>  an antisense primer used for amplifying the sequence of syntaxin
       1a by PCR

<400>  7
ccgctcgagt tacgccttgc tctggtactt                                     30